# EUROPEAN PATENT APPLICATION

(11) **EP 1 862 184 A1**
(43) Date of publication of application: **05.12.2007**
(21) Application number: 06714818.9
(22) Date of filing: 28.02.2006
(51) Int. Cl.: A61K 47/26, A61K 9/20, A61K 47/02, A61K 47/04, A61K 47/32, A61K 47/36, A61K 47/38, A61K 31/5383, A61P 31/04

(54) **PHARMACEUTICAL COMPOSITION**

(30) Priority: 24.03.2005 JP 2005087351
(71) Applicant: Daiichi Sankyo Company, Limited, Chuo-ku Tokyo 103-8426 (JP)
(72) Inventor: NAKAGAMI, Hiroaki, Edogawa-ku, Tokyo 1348630 (JP); KUNO, Yoshio, Edogawa-ku, Tokyo 1348630 (JP)
(74) Representative: Hartz, Nikolai
(86) International application number: PCT/JP2006/303681
(87) International publication number: WO 2006/100875

(57) **Abstract**

The present invention provides an intraoral rapid-disintegrating pharmaceutical composition which exhibits excellent intraoral disintegrating property and high hardness, and which can be applied, in particular, to a pharmaceutically active ingredient which is unstable in water. The pharmaceutical composition, which contains lactose and powdered cellulose, exhibits high hardness without prolongation of its intraoral disintegration time. When a disintegrating agent is further incorporated into the pharmaceutical composition, the hardness of the composition is increased without prolongation of its intraoral disintegration time. When magnesium aluminometasilicate, and one or more species selected from among silicic acid and silicic acid salts other than magnesium aluminometasilicate are further incorporated into the pharmaceutical composition, the composition exhibits higher hardness without prolongation of its intraoral disintegration time. The pharmaceutical composition of the present invention can be produced by means of a generally employed industrial production process without using a special preparation technique.

## Description

### [Technical Field]

The present invention relates to a pharmaceutical composition; and more particularly to a pharmaceutical composition which, when taken in the oral cavity, is rapidly disintegrated by saliva or a small amount of water (hereinafter such a pharmaceutical composition may be referred to as an "intraoral rapid-disintegrating pharmaceutical composition"), particularly, to an intraoral rapid-disintegrating pharmaceutical composition which is useful and has a sufficient hardness against disintegration during usual production, transportation, or use thereof.

### [Background Art]

In recent years, in accordance with transition to an aging society and changes in living environments, a demand has arisen for development of a pharmaceutical preparation which can be readily handled and taken by the elderly, children, or patients whose intake of water is restricted. A preparation which can be taken without water (i.e., a preparation which can be taken anywhere) is useful for the case of, for example, acute onset of a symptom. Thus, attempts have been made to develop an intraoral rapid-disintegrating preparation which, when taken in the oral cavity, is rapidly disintegrated and dissolved merely by saliva or a small amount of water.

For example, there has been developed "Zydis fast-dissolving tablet" (intraorally dissolving preparation), which is commercially available from R. P. Scherer; and there has been disclosed "Intraorally Disintegrating Preparation and Production Method therefor" (see Patent Document 1). This patent document describes an intraoral rapid-disintegrating preparation produced through the following procedure: a solution containing a dissolved or suspended pharmaceutically active ingredient is charged into pockets of a PTP (press through package) sheet which has been molded in advance, and the sheet is subjected to freeze-drying or reduced-pressure drying.
"Rapidly Disintegrating Tablet Containing Polyvinyl Alcohol" (see Patent Document 2) discloses an intraoral rapid-disintegrating preparation produced through the following procedure: a pharmaceutically active ingredient is mixed with a sugar, the resultant mixture is kneaded with an organic solvent or water in which polyvinyl alcohol has been dissolved, and the resultant mixture is charged into a mold and then subjected to low-pressure compression molding via a film, followed by drying. "Intraorally Soluble Compressed Molding and Process for Producing the Same" (see Patent Document 3) discloses an intraorally dissolving compressed product produced by granulating a sugar of low moldability with a sugar of high moldability, and drying the resultant granules, followed by tableting of the granules. "Tablets Quickly Disintegrating in the Oral Cavity and Process for Producing the Same" (see Patent Document 4) discloses an intraoral rapid-disintegrating tablet produced through the following procedure: a pharmaceutically active ingredient, a sugar, and a sugar which becomes amorphous are mixed together; the resultant mixture is formed into granules; and the granules are subj ected to compression molding, followed by humidification and drying.
"Rapidly Disintegrating Solid Preparation" (see Patent Document 5) discloses a rapidly disintegrating solid preparation containing an active ingredient, sugar or sugar alcohol particles having an average particle size of 30 µm to 300 µm, a disintegrant, and a cellulose.
"Medicinal Compositions Quickly Disintegrating in the Oral Cavity and Process for Producing the Same" (see Patent Document 6) discloses an intraoral rapid-disintegrating pharmaceutical composition produced through the following procedure: a sugar and/or sugar alcohol having a high melting point is mixed with a sugar and/or sugar alcohol having a low melting point, the resultant mixture is subjected to compression treatment, and the resultant product is heated at a temperature around the melting point of the sugar and/or sugar alcohol of low melting point.
[Patent Document 1] JP-B-2807346
[Patent Document 2] International Publication WO 01/064190 pamphlet
[Patent Document 3] International Publication WO 95/20380 pamphlet
[Patent Document 4] International Publication WO 99/47124 pamphlet
[Patent Document 5] JP-A-2001-58944
[Patent Document 6] International Publication WO 2002/032403 pamphlet

### [Disclosure of the Invention]

### [Problems to be Solved by the Invention]

The aforementioned Zydis fast-dissolving tablet (intraorally dissolving preparation) exhibits excellent disintegrating property. However, this fast-dissolving tablet has drawbacks, in that the tablet does not have sufficiently high hardness and tends to suffer cracking or chipping, and that production of the tablet requires freeze-drying or reduced-pressure drying, leading to an increase in production cost.
The technique described in JP-B-2807346 (Patent Document 1) "Intraorally Disintegrating Preparation and Production Method therefor" or in International Publication WO 01/064190 pamphlet (Patent Document 2) "Rapidly Disintegrating Tablet Containing Polyvinyl Alcohol" has drawbacks in that, for example, the production method, which employs a suspension or emulsion containing a pharmaceutically active ingredient, has a difficulty applying to a pharmaceutical composition containing a pharmaceutically active ingredient which is unstable in water.
The "rapidly disintegrating solid preparation" described in JP-A-2001-58944 (Patent Document 5) has hardness and intraoral disintegrating property which do not reach the levels described below by the present inventors; i.e., the hardness and intraoral disintegrating property of the solid preparation are not satisfactorily high.
The production method described in International Publication WO 95/20380 pamphlet (Patent Document 3) "Soluble Compressed Molding and Process for Producing the Same," in International Publication WO 99/47124 pamphlet (Patent Document 4) "Tablets Quickly Disintegrating in the Oral Cavity and Process for Producing the Same," or in International Publication WO 2002/032403 pamphlet (Patent Document 6) "Medicinal Compositions Quickly Disintegrating in the Oral Cavity and Process for Producing the Same" has drawbacks in that the production method requires intricate processes and is unsuitable for industrial use, although a product produced through the method exhibits excellent Intraoral disintegrating property and high hardness.
In view of the foregoing, the present inventors have studied on a method for producing an intraoral rapid-disintegrating pharmaceutical composition by means of a generally employed industrial production process without using a special preparation technique, which composition exhibits excellent intraoral disintegrating property and high hardness, and which can be applied, in particular, to a pharmaceutically active ingredient which is unstable in water.

### [Means for Solving the Problems]

The present inventors have conducted extensive studies and as a result have found that a pharmaceutical composition containing lactose and powdered cellulose exhibits excellent intraoral disintegrating property and sufficiently high hardness against disintegration during usual transportation or use thereof.
The present inventors have also found that addition of a disintegrant enables production of anintraoral rapid-disintegrating pharmaceutical composition exhibiting more excellent intraoral disintegratiog property and high hardness.
The present inventors have also found that, surprisingly, when magnesium aluminometasilicate (which is known as a glidant or an antacid agent) is incorporated singly, or in combination with one or more species selected from among silicic acid and silicic acid salts other than magnesium aluminometasilicate, the resultant pharmaceutical composition exhibits increased hardness without prolongation of the disintegration time thereof. The present invention has been accomplished on the basis of these findings.

Accordingly, the present invention provides the following:
1. A pharmaceutical composition comprising a lactose and a powdered cellulose;
2. The pharmaceutical composition according to 1, which further comprises a disintegrant;
3. The pharmaceutical composition according to 2, wherein the disintegrant is one or more species selected from among low-substituted hydroxypropyl cellulose, crospovidone, sodium carboxymethyl starch, carmellose, and sodium croscarmellose;
4. The pharmaceutical composition according to 2 or 3, wherein the disintegrant is crospovidone and/or carmellose;
5. The pharmaceutical composition according to any one of 1 through 4, which further comprises a magnesium aluminometasilicate;
6. The pharmaceutical composition according to any one of 1 through 5, which further comprises one or more species selected from among silicic acid and silicic acid salts other than magnesium aluminometasilicate;
7. The pharmaceutical composition according to 6, wherein the one or more species selected from among silicic acid and silicic acid salts other than magnesium aluminometasilicate are calcium silicate and/or light anhydrous silicic acid;
8. The pharmaceutical composition according to any one of 1 through 7, which further comprises a pharmaceutically active ingredient;
9. The pharmaceutical composition according to any one of 1 through 8, wherein the total amount of the lactose and the powdered cellulose is 5 to 99.5 w/w% on the basis of the entire amount of the pharmaceutical composition;
10. The pharmaceutical composition according to any one of 1 through 9, wherein the amount of the powdered cellulose is 1 to 100 parts by weight on the basis of 100 parts by weight of the lactose;
11. The pharmaceutical composition according to any one of 2 through 10, wherein the amount of the disintegrant is 0.1 to 15 w/w% on the basis of the entire amount of the pharmaceutical composition;
12. The pharmaceutical composition according to any one of 5 through 11, wherein the amount of the magnesium aluminometasilicate is 0.05 to 5 w/w% on the basis of the entire amount of the pharmaceutical composition;
13. The pharmaceutical composition according to any one of 6 through 11, wherein the amount of the one or more species selected from among silicic acid and silicic acid salts other than magnesium aluminometasilicate is 0.05 to 5 w/w% on the basis of the entire amount of the pharmaceutical composition;
14. The pharmaceutical composition according to any one of 6 through 13, wherein the amount of the one or more species selected from among silicic acid and silicic acid salts other than magnesium aluminometasilicate is 0.01 to 20 parts by weight on the basis of 1 part by weight of the magnesium aluminometasilicate;
15. The pharmaceutical composition according to any one of 7 through 13, wherein the amount of the calcium silicate and/or the light anhydrous silicic acid is 0.01 to 20 parts by weight on the basis of 1 part by weight of the magnesium aluminometasilicate;
16. The pharmaceutical composition according to any one of 8 through 15, wherein the amount of the pharmaceutically active ingredient is 0.01 to 80 w/w% on the basis of the entire amount of the pharmaceutical composition;
17. The pharmaceutical composition according to any one of 1 through 16, which exhibits rapid disintegrating property;
18. The pharmaceutical composition according to any one of 1 through 16, which exhibits intraoral rapid disintegrating property;
19. The pharmaceutical composition according to any one of 1 through 18, which takes a form of tablet;
20. A pharmaceutical composition comprising a magnesium aluminometasilicate, and one or more species selected from among silicic acid and silicic acid salts other than magnesium aluminometasilicate;
21. The pharmaceutical composition according to 20, wherein the one or more species selected from among silicic acid and silicic acid salts other than magnesium aluminometasilicate are calcium silicate and/or light anhydrous silicic acid;
22. A method for increasing the hardness of a tablet, comprising adding a magnesium aluminometasilicate to a tablet;
23. The method according to 22, which further comprises adding, to the tablet, one or more species selected from among silicic acid and silicic acid salts other than magnesium aluminometasilicate; and
24. A method for increasing the hardness of a tablet, comprising adding, to a tablet, a magnesium aluminometasilicate, and a calcium silicate and/or a light anhydrous silicic acid.
25. Use of the pharmaceutical composition according to any one of 1 through 16, for manufacturing an intraoral rapid disintegrating preparation.
26. Use according to 25, wherein the intraoral rapid disintegrating preparation is an intraoral rapid disintegrating tablet.

### [Effects of the Invention]

As is clear from the below-described Examples, the pharmaceutical composition of the present invention is rapidly disintegrated in the oral cavity, and has a hardness sufficient for practical use. Therefore, the pharmaceutical composition of the present invention can be provided in the form of an intraoral rapid-disintegrating tablet which can be readily taken by children, the elderly, or patients who have difficulty in swallowing drugs, or in the form of a product employed for preventing or treating diseases of patients whose intake of water is restricted, or patients in an emergency situation in which water or the like is not readily available. The pharmaceutical composition of the present invention can be produced by means of a generally employed industrial production process. [Best Mode for Carrying Out the Invention]

The pharmaceutical composition of the present invention comprises lactose and powdered cellulose as essential components. A compression product obtained through compression molding of the composition containing these essential components exhibits excellent intraoral disintegrating property and sufficiently high hardness against disintegration during usual transportation or use thereof.

When the pharmaceutical composition of the present invention is taken in the oral cavity of a healthy male adult without using water, the time required for the composition to completely disintegrate or dissolve in the oral cavity (hereinafter the time may be referred to as an "intraoral disintegration time") is generally 90 seconds or less, preferably 60 seconds or less, more preferably 30 seconds or less, much more preferably 15 seconds or less. When the pharmaceutical composition is taken by a subject in a general manner, since the composition is licked with the tongue of the subject or is pressed by the tongue against the upper jaw, the composition is disintegrated within a shorter period of time. The pharmaceutical composition of the present invention has a hardness sufficient for practical use; i.e., generally 2 kp or more, preferably 3 kp or more, more preferably 4 kp or more.

In the pharmaceutical composition of the present invention, the total amount of the lactose and the powdered cellulose is preferably 5 to 99.5 w/w%, more preferably 10 to 90 w/w%, on the basis of the entire amount of the pharmaceutical composition. In the pharmaceutical composition of the present invention, the amount of the powdered cellulose is preferably 1 to 100 parts by weight, more preferably 5 to 50 parts by weight, on the basis of 100 parts by weight of the lactose.

The pharmaceutical composition of the present invention preferably comprises a disintegrant. Examples of the disintegrant include, but are not limited to, one species or combinations of two or more species selected from among, for example, low-substituted hydroxypropyl cellulose, crospovidone, sodium carboxymethyl starch, carmellose, and sodium croscarmellose. In the present invention, crospovidone and/or carmellose is preferred, with crospovidone being more preferred. The amount of the disintegrant may be appropriately determined in accordance with the target hardness or intraoral disintegrating property of the pharmaceutical composition, but the amount of the disintegrant is preferably 0.1 to 15% by weight, more preferably 0.5 to 12% by weight, even more preferably 1 to 10% by weight, on the basis of the entire amount of the pharmaceutical composition of the present invention.

When magnesium aluminometasilicate, and one or more species selected from among silicic acid and silicic acid salts other than magnesium aluminometasilicate (hereinafter may be referred to as "the other silicic acid/silicic acid salts") are added to the pharmaceutical composition of the present invention, a product formed by compressed at a genaral compression pressure exhibits sufficient hardness and satisfactory disintegrating property (i.e. , prolongation of the disintegration time of the compressed product is suppressed). As is generally known, when the compression pressure is increased, the hardness can be increased, but the disintegrating property is impaired, whereas when the compression pressure is reduced, the disintegrating property can be improved, but the hardness is lowered. In order to attain satisfactory disintegrating property and hardness, magnesium aluminometasilicate is preferably employed in combination with the other silicic acid/silicic acid salts, although magnesium aluminometasilicate may be employed singly. Examples of the other silicic acid/silicic acid salts include, but are not limited to, calcium silicate, magnesium silicate, aluminum silicate, magnesium aluminosilicate, aluminum metasilicate, light anhydrous silicic acid, and silicic acid hydrate. These may be employed singly, or in combination of two or more species. In the present invention, more preferably, magnesium aluminometasilicate is employed in combination with calcium silicate and/or light anhydrous silicic acid.

The present invention also provides a pharmaceutical composition which, when formed into a compressed product, exhibits desired disintegrating property and sufficient hardness; specifically, a pharmaceutical composition containing magnesium aluminometasilicate, and one or more species selected from among the other silicic acid/silicic acid salts.
In the present invention, the amount of the magnesium aluminometasilicate may be appropriately determined in accordance with the target hardness or disintegrating property of the pharmaceutical composition, but the amount of the magnesium aluminometasilicate is preferably 0.05 to 5 w/w%, more preferably 0.1 to 4 w/w%, on the basis of the entire amount of the pharmaceutical composition. The amount of one or more species selected from among the other silicic acid/silicic acid salts may be appropriately determined in accordance with the target hardness or disintegrating property of the pharmaceutical composition, but the amount of the thus-selected one or more species is preferably 0.05 to 5 w/w%, more preferably 0.1 to 4 w/w%, on the basis of the entire amount of the pharmaceutical composition. When the magnesium aluminometasilicate is employed in combination with the other silicic acid/silicic acid salts, the amount of the other silicic acid/silicic acid salts is preferably 0.01 to 20 parts by weight, more preferably 1 to 10 parts by weight, on the basis of 1 part by weight of the magnesium aluminometasilicate.

No particular limitations are imposed on the pharmaceutically active ingredient to be incorporated into the pharmaceutical composition of the present invention, so long as the active ingredient can be orally administered. The pharmaceutically active ingredient to be employed is, for example, one or more species selected from among a vitamin, an antipyretic analgesic anti-inflammatory agent, an antihistamine, an antitussive, a gastric mucosa restoring agent, an antipyretic antispastic agent, a psychotropic drug, an antiemetic agent, an antidepressant, an H₂ receptor blocker, a proton pump inhibitor, a chemotherapeutic agent, an antibacterial agent, a hypotensive agent, an arrhythmia treatment agent, an antithrombotic drug, an antirheumatic drug, an anti-anxiety drug, an anti-dementia drug, an ACE inhibitor, and an angiotensin II receptor antagonist. Specific examples of such a pharmaceutically active ingredient include, but are not limited to, thiamine hydrochloride, nicotinamide, ascorbic acid, pantethine, ethenzamide, aspirin, acetaminophen, cetraxate hydrochloride, indomethacin, meloxicam, diphenhydramine hydrochloride, procaterol hydrochloride, meclofenoxate hydrochloride, lorazepam, phenobarbital, timiperone, calcium p-aminosalicylate, ampicillin, carmofur, levofloxacin, ofloxacin, nifedipine, carvedilol, procainamide hydrochloride, ticlopidine hydrochloride, cevimeline hydrochloride hydrate, alimemazine tartrate, lofepramine hydrochloride, isoniazid, baclofen, cetirizine hydrochloride, isoxsuprine hydrochloride, N-methylscopolamine methylsulfate, trihexyphenidyl hydrochloride, oxypertine, memantine hydrochloride, captopril, and perindopril erbumine.

The pharmaceutical composition of the present invention can be produced without using water. Therefore, a pharmaceutically active ingredient which is unstable in water is particularly suitable for use in the pharmaceutical composition of the present invention.
As used herein, the expression "pharmaceutically active ingredient which is unstable in water" refers to a pharmaceutically active ingredient which, when stored at 25°C in 75% RH for three months, decreases by 5% or more of its initial content.
Examples of such a pharmaceutically active ingredient which is unstable in water include, but are not limited to, thiamine hydrochloride, nicotinamide, aspirin, acetaminophen, indomethacin, diphenhydramine hydrochloride, procaterol hydrochloride, meclofenoxate hydrochloride, lorazepam, phenobarbital, calcium p-aminosalicylate, ampicillin, carmofur, captopril, nifedipine, procainamide hydrochloride, and perindopril erbumine.

In the present invention, the amount of the pharmaceutically active ingredient is generally 0.01 to 80 w/w%, preferably 0.1 to 60 w/w%. The amount of the pharmaceutically active ingredient may be varied within a range such that the effects of the present invention are not impeded.

The pharmaceutical composition of the present invention may contain, in addition to the aforementioned essential components, a known additive employed for drug preparation, such as an excipient, a binder, a disintegrant, a lubricant, a coloring agent, or a flavoring agent. Preferably, the amount of such an additive is regulated to a level or extent such that the effects of the present invention are not impeded.

In the present invention, preferably, the excipient is preferably one species or a combination of two or more species selected from among a sugar, a sugar alcohol, and a cellulose.
Examples of the sugar include, but are not limited to, glucose, fructose, sucrose, anhydrous lactose, and trehalose. Examples of the sugar alcohol include, but are not limited to, mannitol, erythritol, sorbitol, xylitol, and maltitol. Examples of the cellulose include, but are not limited to, microcrystalline cellulose and low-substituted hydroxypropyl cellulose.
Examples of the lubricant include, but are not limited to, magnesium stearate, sodium stearylfumarate, sucrose esters of fatty acids, hydrogenated oils, and talc.

Preferred formulation of the pharmaceutical composition of the present invention is tablet. The pharmaceutical composition of the present invention can be used for manufacturing a rapid disintegrating preparation, preferably a rapid disintegrating tablet, more preferably an intraoral rapid disintegrating preparation, still more preferably an intraoral rapid disintegrating tablet.

Next will be described an example of a method for producing the pharmaceutical composition of the present invention.
The pharmaceutical composition of the present invention can be produced by means of a generally known tablet production method by use of a generally employed drug production apparatus.

All or a portion of the components of the pharmaceutical composition of the present invention; i.e., a pharmaceutically active ingredient, lactose, powdered cellulose, and other additives for drug preparation which are employed if desired (e.g., magnesium aluminometasilicate, the other silicic acid/silicic acid salts, and a disintegrant), is subjected to milling if necessary, and the thus-milled product is subjected to mixing. Milling of the components can be performed by use of, for example, a hammer mill, a cutting mill, a rotary mill, a fluid energy mill, a screening mill, or a tumbler mill. Mixing of the thus-milled product can be performed by use of a V-type mixer, a double-cone mixer, a high-speed mixer, or a Nauta mixer.

If desired, an additive for drug preparation may be added during the course of mixing, which may be followed by granulation by use of, for example, a fluidized bed granulator, a wet shear granulator, a tumbling granulator, or an extrusion granulator. Generally employed granulation techniques include wet granulation, dry granulation, and melt granulation, any of which maybe employed in the present invention. When a pharmaceutically active ingredient which is unstable in water is applied to the present invention, preferably, dry granulation is carried out. Examples of the wet granulation technique include fluidized bed granulation, mixing wet shear granulation, extrusion granulation, and tumbling granulation. The dry granulation technique is a technique for directly granulating all or a portion of the components of the pharmaceutical composition of the present invention; i.e., a pharmaceutically active ingredient, lactose, powdered cellulose, and other additives for drug preparation which are employed if desired (e.g., magnesium aluminometasilicate, the other silicic acid/silicic acid salts, and a disintegrating agent). The melt granulation technique is a technique for granulating the components through melting thereof under heating. In the present invention, any of these granulation techniques may be carried out.

The thus-prepared mixture and/or granules can be subjected to compression treatment by use of a generally employed tablet forming machine, such as a single-stroke tableting machine, a rotary tableting machine, or an external-lubrication tableting machine.

The compression pressure during the compression treatment may be determined in accordance with the target hardness of a compressed-product, or the disintegrating property or solubility thereof when taken in the oral cavity.
The compression pressure is generally 100 to 2,000 kgf, preferably 200 to 1,800 kgf, more preferably about 300 to about 1,500 kgf.

### [Examples]

The present invention will next be described in detail by way of Examples, which should not be construed as limiting the invention thereto.

### (Test methods)

In order to describe the effects of the present invention in more detail, tablets produced in Test Examples and Examples were subjected to testing for evaluation of the following characteristics.

### (Hardness test)

The fracture strength of a tablet in its radial direction was measured by use of a tablet hardness meter (product of Elbaker).

### (Intraoral disintegration test)

A tablet was taken in the oral cavity of a healthy male adult without water, and the time required for the tablet to disintegrate and dissolve in the oral cavity (i.e., intraoral disintegration time) was measured.

### (Example 1)

Powdered cellulose (ARBOCEL M-80, RETTENMAIER & SOHNE) (109.1 g), calcium silicate (Florite-RE, Tokuyama) (27.3 g), magnesium aluminometasilicate (Neusilin UFL2, Toyama Chemical Co., Ltd.) (9.1 g), and crospovidone (Polyplasdone-XL, ISP. JAPAN) (27.3 g) were placed in a high-speed mixer (FS-5J, Fukae Powtec Co., Ltd.), and were mixed together at 480 rpm for three minutes, to thereby prepare a powder mixture A (172.8 g). Separately, powdered cellulose (9. 0 g) and red ferric oxide (Kishi Kasei Co., Ltd.) (0.9 g) were mixed together by use of a tablet mill (model: KC-HUK, Konishi Seisakusho Co. , Ltd.) for one minute, to thereby prepare a powder mixture B. The thus-prepared powder mixture B (9.9 g), perindopril erbumine (Servier) (18.2 g), Orange Micron (Takasago International Corporation) (0.9 g), and the powder mixture A (172.8 g) were mixed together by use of a high-speed mixer at 600 rpm for three minutes, to thereby prepare a powder mixture C (201.8 g). Subsequently, the powder mixture C (201.8 g) and lactose (Lactose Monohydrate, Pharmatose 100M, DMV) (797.3 g) were placed in a V-type mixer (UM-V-5, Kawagoe Kikai), and were mixed together for 20 minutes, to thereby prepare a powder mixture D. The thus-prepared powder mixture D was subjected to tableting by use of an external-lubrication tableting machine (VIRG 0512SS2, AZ tableting machine, Kikusui Seisakusho Ltd.) (tableting pressure: 800 kgf, punch size: 7.0 mmφ), to thereby yield tablets, each having a weight of 110 mg (intraorally disintegrating pharmaceutical composition). In this case, magnesium stearate (Nitto Chemical Industry Co. , Ltd.) was employed as a lubricant.

### (Example 2)

Powdered cellulose (108.0 g), calcium silicate (26.7 g), magnesium aluminometasilicate (10.0 g), and crospovidone (26.7 g) were placed in a high-speed mixer, and were mixed together at 480 rpm for three minutes, to thereby prepare a powder mixture E (171.4 g). Separately, powdered cellulose (10.0 g), yellow ferric oxide (Kishi Kasei Co., Ltd.) (0.5 g), and red ferric oxide (0.5 g) were mixed together by use of a tablet mill, to thereby prepare a powder mixture F. The thus-prepared powder mixture F (11.0 g), perindopril erbumine (26.7 g), Orange Micron (1.0 g), and the powder mixture E (171.4 g) were mixed together by use of a high-speed mixer at 600 rpm for three minutes, to thereby prepare a powder mixture G (210.1 g). Subsequently, the powder mixture G (210.1 g) and lactose (789.0 g) were placed in a V-type mixer, and were mixed together for 20 minutes, to thereby prepare a powder mixture H. The thus-prepared powder mixture H was subjected to tableting by use of an external-lubrication tableting machine (tableting pressure: 800 kgf, punch size: 7.5 mmφ), to thereby yield tablets, each having a weight of 150 mg (intraorally disintegrating pharmaceutical composition). In this case, magnesium stearate was employed as a lubricant.

**Table 1**

| | Example 1 | Example 2 |
|---|---|---|
| Intraoral disintegration time (seconds) | 14 | 15 |
| Hardness (kp) | 4.2 | 4.3 |

Each of the pharmaceutical compositions of Examples 1 and 2 (i.e., the pharmaceutical composition of the present invention) was subjected to measurement in terms of intraoral disintegration time and hardness. The results are shown in Table 1. Each of the intraoral rapid-disintegrating pharmaceutical compositions of Examples 1 and 2 was found to exhibit desirable characteristics (i.e., an intraoral disintegration time of 15 seconds or less, and a hardness of 4.0 kp or more).

### (Test Example 1)

### (Test Example 1.1)

Microcrystalline cellulose (220 g), low-substituted hydroxypropyl cellulose (LH-11, Shin-Etsu Chemical Co., Ltd.) (55 g), hydroxypropyl cellulose (HPC-L, Shin-Etsu Chemical Co., Ltd.) (33 g), and light anhydrous silicic acid (2 g) were mixed together by use of a high-speed mixer for five minutes. Subsequently, anhydrous lactose (DCL-21, DMV) (784.5 g) and magnesium stearate (5.5 g) were added to the resultant mixture, and were mixed together by use of a V-type mixer for 30 minutes. The thus-prepared powder mixture was subjected to continuous tableting by use of a rotary tableting machine (VIRG 0512SS2, AZ tableting machine, Kikusui Seisakusho Ltd.) (tableting pressure: 900 kgf, punch size : 7.0 mmφ), to thereby yield tablets, each having a weight of 110 mg.

### (Test Example 1.2)

The procedure of Test Example 1.1 was repeated, except that the anhydrous lactose was replaced by lactose, to thereby yield tablets.

### (Test Example 1.3)

The procedure of Test Example 1.1 was repeated, except that the anhydrous lactose was replaced by mannitol (PEARLITOL 200SD, ROQUETTE), to thereby yield tablets.

### (Test Example 1.4)

The procedure of Test Example 1.1 was repeated, except that the anhydrous lactose was replaced by sucrose (SUCRE COMPRESSUC MS, Beghin Say), to thereby yield tablets.

### (Test Example 1.5)

The procedure of Test Example 1.1 was repeated, except that the anhydrous lactose was replaced by erythritol (fine powder grade, Nikken Chemicals Co., Ltd.), to thereby yield tablets.

### (Test Example 1.6)

The procedure of Test Example 1.1 was repeated, except that the anhydrous lactose was replaced by trehalose (Hayashibara), to thereby yield tablets.

### (Test Example 1.7)

The procedure of Test Example 1.1 was repeated, except that the anhydrous lactose was replaced by sorbitol (Sorbit DP-10M, Towa Chemical Industry Co., Ltd.), to thereby yield tablets.

Table 2 shows the results of the above-described Test Examples, which employ different primary excipients.

**Table 2**

| | Hardness (kp) | Intraoral disintegration time (seconds) |
|---|---|---|
| Test Example 1.1 | 9.8 | 240 |
| Test Example 1.2 | 4.8 | 31 |
| Test Example 1.3 | 7.1 | 50 |
| Test Example 1.4 | 6.9 | 138 |
| Test Example 1.5 | 1.9 | 11 |
| Test Example 1.6 | 3.8 | 124 |
| Test Example 1.7 | 7.0 | 242 |

The results of the above Test Examples shown in Table 2, which employ various sugars and sugar alcohols, indicate that a tablet containing lactose exhibits short intraoral disintegration time and high hardness.

### (Test Example 2)

### (Test Example 2.1)

Calcium silicate (20.0 g), magnesium aluminometasilicate (10.0 g), and hydroxypropyl cellulose (33 g) were mixed together by use of a high-speed mixer for five minutes. Subsequently, microcrystalline cellulose (190 g) and low-substituted hydroxypropyl cellulose (55 g) were added to the resultant mixture, and were mixed together by use of a V-type mixer for five minutes. Thereafter, lactose (786.5 g) and magnesium stearate (5.5 g) were added to the resultant mixture, and were mixed together by use of the V-type mixer for 30 minutes, to thereby prepare a powder mixture. The thus-prepared powder mixture was subjected to continuous tableting by use of a rotary tableting machine (tableting pressure: 900 kgf, punch size: 7.0 mmφ), to thereby yield tablets, each having a weight of 110 mg.

### (Test Example 2.2)

The procedure of Test Example 2.1 was repeated, except that the low-substituted hydroxypropyl cellulose was replaced by crospovidone, to thereby yield tablets.

### (Test Example 2.3)

The procedure of Test Example 2.1 was repeated, except that the low-substituted hydroxypropyl cellulose was replaced by sodium carboxymethyl starch (Primojel, Matsutani Chemical Industry Co., Ltd.), to thereby yield tablets.

### (Test Example 2.4)

The procedure of Test Example 2. 1 was repeated, except that the low-substituted hydroxypropyl cellulose was replaced by carmellose (NS-300, Gotoku Chemical Company Ltd.), to thereby yield tablets.

### (Test Example 2.5)

The procedure of Test Example 2.1 was repeated, except that the low-substituted hydroxypropyl cellulose was replaced by croscarmellose sodium (Ac-Di-Sol, Asahi Kasei Corporation), to thereby yield tablets.

Table 3 shows the results of the above-described Test Examples, which employ different disintegrating agents.

**Table 3**

| | Hardness (kp) | Intraoral disintegration time (seconds) |
|---|---|---|
| Test Example 2.1 | 5.3 | 46 |
| Test Example 2.2 | 4.4 | 17 |
| Test Example 2.3 | 4.6 | 28 |
| Test Example 2.4 | 4.9 | 19 |
| Test Example 2.5 | 4.7 | 30 |

The results of the above Test Examples shown in Table 4, which employ various disintegrating agents, indicate that a tablet containing crospovidone or carmellose exhibits short intraoral disintegration time and high hardness.

### (Test Example 3)

### (Test Example 3.1)

Calcium silicate (20.0 g), magnesium aluminometasilicate (10.0 g), and hydroxypropyl cellulose (33 g) were mixed together by use of a high-speed mixer for five minutes. Subsequently, microcrystalline cellulose (190 g) and low-substituted hydroxypropyl cellulose (55 g) were added to the resultant mixture, and were mixed together by use of a V-type mixer for five minutes. Thereafter, lactose (786.5 g) and magnesium stearate (5.5 g) were added to the resultant mixture, and were mixed together by use of the V-type mixer for 30 minutes, to thereby prepare a powder mixture. The thus-prepared powder mixture was subjected to continuous tableting by use of a rotary tableting machine (tableting pressure: 900 kgf punch size 7. 0 mmφ), to thereby yield tablets, each having a weight of 110 mg.

### (Test Example 3.2)

The procedure of Test Example 3.1 was repeated, except that the microcrystalline cellulose was replaced by powdered cellulose, to thereby yield tablets.

### (Test Example 3.3)

The procedure of Test Example 3.1 was repeated, except that the microcrystalline cellulose was replaced by partly pregelatinized starch (PCS, Asahi Kasei Corporation), to thereby yield tablets.

### (Test Example 3.4)

The procedure of Test Example 3.1 was repeated, except that the microcrystalline cellulose was replaced by partly pregelatinized starch (Starch 4500, Colorcon Japan), to thereby yield tablets.

### (Test Example 3.5)

The procedure of Test Example 3.1 was repeated, except that the microcrystalline cellulose was replaced by dibasic calcium phosphate (Nitto Kagaku), to thereby yield tablets.

Table 4 shows the results of the above-described Test Examples, which employ different secondary excipients.

**Table 4**

| | Hardness (kp) | Intraoral disintegration time (seconds) |
|---|---|---|
| Test Example 3.1 | 4.4 | 17 |
| Test Example 3.2 | 6.9 | 15 |
| Test Example 3.3 | 3.0 | 47 |
| Test Example 3.4 | 3.4 | 47 |
| Test Example 3.5 | 3.2 | 14 |

The results of the above Test Examples, which employ various excipients, show that a tablet containing lactose in combination with powdered cellulose exhibits short intraoral disintegration time and high hardness.

### (Test Example 4.1)

Powdered cellulose (115.9 g), calcium silicate (27.3 g), magnesium aluminometasilicate (9.1 g), and crospovidone (27.3 g) were placed in a high-speed mixer, and were mixed together at 480 rpm for three minutes, to thereby prepare a powder mixture I (179.6 g). Separately, powdered cellulose (2.3 g) and red ferric oxide (0.2 g) were mixed together by use of a tablet mill, to thereby prepare a powder mixture J. The thus-prepared powder mixture J (2.5 g), Orange Micron (0.9 g), and the powder mixture I (179.6 g) were mixed together by use of a high-speed mixer at 600 rpm for three minutes, to thereby prepare a powder mixture K. Subsequently, the powder mixture K (183.0 g) and lactose (816.1 g) were placed in a V-type mixer, and were mixed together for 20 minutes, to thereby prepare a powder mixture L. The thus-prepared powder mixture L was subjected to tableting by use of an external-lubrication tableting machine (tableting pressure: 600 kgf, punch size: 7. 0 mmφ), to thereby yield tablets, each having a weight of 110 mg (intraorally disintegrating pharmaceutical composition). In this case, magnesium stearate was employed as a lubricant.

### (Test Example 4.2)

The procedure of Test Example 4.1 was repeated, except that the tableting pressure was changed to 700 kgf, to thereby yield tablets (intraorally disintegrating pharmaceutical composition).

### (Test Example 4.3)

The procedure of Test Example 4.1 was repeated, except that the tableting pressure was changed to 800 kgf, to thereby yield tablets (intraorally disintegrating pharmaceutical composition).

### (Test Example 4.4)

The procedure of Test Example 4.1 was repeated, except that the tableting pressure was changed to 900 kgf, to thereby yield tablets (intraorally disintegrating pharmaceutical composition).

### (Test Example 4.5)

The procedure of Test Example 4.1 was repeated, except that the tableting pressure was changed to 1, 000 kgf, to thereby yield tablets (intraorally disintegrating pharmaceutical composition).

### (Test Example 4.6)

The procedure of Test Example 4.1 was repeated, except that the tableting pressure was changed to 1, 100 kgf, to thereby yield tablets (intraorally disintegrating pharmaceutical composition).

### (Test Example 4.7)

Powdered cellulose (115.9 g) and crospovidone (27.3 g) were placed in a high-speed mixer, and were mixed together at 480 rpm for three minutes, to thereby prepare a powder mixture M (143.2 g) . Separately, powdered cellulose (2.3 g) and red ferric oxide (0.2 g) were mixed together by use of a tablet mill, to thereby prepare a powder mixture N. The thus-prepared powder mixture N (2.5 g), Orange Micron (0.9 g), and the powder mixture M (143.2 g) were mixed together by use of a high-speed mixer at 600 rpm for three minutes, to thereby prepare a powder mixture O. Subsequently, the powder mixture O (146.6 g) and lactose (852.5 g) were placed in a V-type mixer, and were mixed together for 20 minutes, to thereby prepare a powder mixture P. The thus-prepared powder mixture P was subjected to tableting by use of an external-lubrication tableting machine (tableting pressure: 600 kgf, punch size: 7.0 mmφ), to thereby yield tablets, each having a weight of 110 mg (intraorally disintegrating pharmaceutical composition). In this case, magnesium stearate was employed as a lubricant.

### (Test Example 4.8)

The procedure of Test Example 4.7 was repeated, except that the tableting pressure was changed to 700 kgf, to thereby yield tablets (intraorally disintegrating pharmaceutical composition).

### (Test Example 4.9)

The procedure of Test Example 4.7 was repeated, except that the tableting pressure was changed to 800 kgf, to thereby yield tablets (intraorally disintegrating pharmaceutical composition).

### (Test Example 4.10)

The procedure of Test Example 4.7 was repeated, except that the tableting pressure was changed to 900 kgf, to thereby yield tablets (intraorally disintegrating pharmaceutical composition).

### (Test Example 4.11)

The procedure of Test Example 4.7 was repeated, except that the tableting pressure was changed to 1, 000 kgf, to thereby yield tablets (intraorally disintegrating pharmaceutical composition).

### (Test Example 4.12)

The procedure of Test Example 4.7 was repeated, except that the tableting pressure was changed to 1,100 kgf, to thereby yield tablets (intraorally disintegrating pharmaceutical composition).

### (Test Example 4.13)

Powdered cellulose (115.9 g), magnesium aluminometasilicate (9.1 g), and crospovidone (27.3 g) were placed in a high-speed mixer, and were mixed together at 480 rpm for three minutes, to thereby prepare a powder mixture Q (152.3 g). Separately, powdered cellulose (2.3 g) and red ferric oxide (0.2 g) were mixed together by use of a tablet mill, to thereby prepare a powder mixture R. The thus-prepared powder mixture R (2.5 g), Orange Micron (0.9 g), and the powder mixture Q (152.3 g) were mixed together by use of a high-speed mixer at 600 rpm for three minutes, to thereby prepare a powder mixture S. Subsequently, the powder mixture S (155.7 g) and lactose (843.4 g) were placed in a V-type mixer, and were mixed together for 20 minutes, to thereby prepare a powder mixture T. The thus-prepared powder mixture T was subjected to tableting by use of an external-lubrication tableting machine (tableting pressure: 600 kgf, punch size: 7.0 mmφ) , to thereby yield tablets, each having a weight of 110 mg (intraorally disintegrating pharmaceutical composition). In this case, magnesium stearate was employed as a lubricant.

### (Test Example 4.14)

The procedure of Test Example 4.13 was repeated, except that the tableting pressure was changed to 700 kgf, to thereby yield tablets (intraorally disintegrating pharmaceutical composition).

### (Test Example 4.15)

The procedure of Test Example 4 . 13 was repeated, except that the tableting pressure was changed to 800 kgf, to thereby yield tablets (intraorally disintegrating pharmaceutical composition).

### (Test Example 4.16)

The procedure of Test Example 4.13 was repeated, except that the tableting pressure was changed to 900 kgf, to thereby yield tablets (intraorally disintegrating pharmaceutical composition).

### (Test Example 4.17)

The procedure of Test Example 4.13 was repeated, except that the tableting pressure was changed to 1,000 kgf, to thereby yield tablets (intraorally disintegrating pharmaceutical composition) .

### (Test Example 4.18)

The procedure of Test Example 4.13 was repeated, except that the tableting pressure was changed to 1,100 kgf, to thereby yield tablets (intraorally disintegrating pharmaceutical composition).

### (Test Example 4.19)

Powdered cellulose (115.9 g), calcium silicate (27.3 g), and crospovidone (27.3 g) were placed in a high-speed mixer, and were mixed together at 480 rpm for three minutes, to thereby prepare a powder mixture U (170.5 g). Separately, powdered cellulose (2.3 g) and red ferric oxide (0.2 g) were mixed together by use of a tablet crusher, to thereby prepare a powder mixture V. The thus-prepared powder mixture V (2. 5 g), Orange Micron (0. 9 g), and the powder mixture U (170.5 g) were mixed together by use of a high-speed mixer at 600 rpm for three minutes, to thereby prepare a powder mixture W. Subsequently, the powder mixture W (173.9 g) and lactose (825.2 g) were placed in a V-type mixer, and were mixed together for 20 minutes, to thereby prepare a powder mixture X. The thus-prepared powder mixture X was subjected to tableting by use of an external-lubrication tableting machine (tableting pressure: 600 kgf, punch size: 7.0 mmφ), to thereby yield tablets, each having a weight of 110 mg (intraorally disintegrating pharmaceutical composition). In this case, magnesium stearate was employed as a lubricant.

### (Test Example 4.20)

The procedure of Test Example 4.19 was repeated, except that the tableting pressure was changed to 700 kgf, to thereby yield tablets (intraorally disintegrating pharmaceutical composition).

### (Test Example 4.21)

The procedure of Test Example 4.19 was repeated, except that the tableting pressure was changed to 800 kgf, to thereby yield tablets (intraorally disintegrating pharmaceutical composition).

### (Test Example 4.22)

The procedure of Test Example 4 . 19 was repeated, except that the tableting pressure was changed to 900 kgf, to thereby yield tablets (intraorally disintegrating pharmaceutical composition).

### (Test Example 4.23)

The procedure of Test Example 4.19 was repeated, except that the tableting pressure was changed to 1, 000 kgf, to thereby yield tablets (intraorally disintegrating pharmaceutical composition).

### (Test Example 4.24)

The procedure of Test Example 4.19 was repeated, except that the tableting pressure was changed to 1,100 kgf, to thereby yield tablets (intraorally disintegrating pharmaceutical composition).

In Test Examples 4.1 through 4.24, the effect of addition of calcium silicate or magnesium aluminometasilicate on the intraoral disintegration time and hardness of the resultant tablets was evaluated at different tableting pressures. The results are shown in Table 5. There was prepared a graph showing the relation between the hardness and intraoral disintegration time of tablets of each of the following groups: a group in which neither calcium silicate nor magnesium aluminometasilicate was added (hereinafter the group will be referred to as "no addition group"); a group in which merely calcium silicate was added (hereinafter the group will be referred to as "calcium silicate group"); a group in which merely magnesium aluminometasilicate was added (hereinafter the group will be referred to as "magnesium aluminometasilicate group"); and a group in which calcium silicate and magnesium aluminometasilicate were added in combination (hereinafter the group will be referred to as "combination group"). Comparison among the aforementioned four groups revealed that, in the case of the tablets of the combination group, prolongation of the intraoral disintegration time associated with an increase in the hardness which occurs as a result of an increase in the tableting pressure-is most suppressed; i.e., the tablets of the combination groups exhibit excellent characteristics.

**Table 5**

| | | Test Ex. 4.1 | Test Ex. 4.2 | Test Ex. 4.3 | Test Ex. 4.4 | Test Ex. 4.5 | Test Ex. 4.6 |
|---|---|---|---|---|---|---|---|
| Combination group | Hardness (kp) | 3.81 | 4.83 | 5.83 | 6.75 | 7.30 | 8.44 |
| | Intraoral disintegration time | 6 | 6 | 7 | 8 | 9 | 10 |
| No addition group | | Test Ex. 4.7 | Test Ex. 4.8 | Test Ex. 4.9 | Test Ex. 4.10 | Test Ex. 4.11 | Test Ex. 4.12 |
| | Hardness (kp) | 4.59 | 5.58 | 6.12 | 6.73 | 7.69 | 8.09 |
| | Intraoral disintegration time | 6 | 7 | 8 | 9 | 11 | 12 |
| Magnesium alumino-metasilicate group | | Test Ex. 4.13 | Test Ex. 4.14 | Test Ex. 4.15 | Test Ex. 4.16 | Test Ex. 4.17 | Test Ex. 4.18 |
| | Hardness (kp) | 3.00 | 3.90 | 4.61 | 5.06 | 5.85 | 6.36 |
| | Intraoral disintegration time | 5 | 6 | 6 | 7 | 8 | 8 |
| Calcium silicate group | | Test Ex. 4.19 | Test Ex. 4.20 | Test Ex. 4.21 | Test Ex. 4.22 | Test Ex. 4.23 | Test Ex. 4.24 |
| | Hardness (kp) | 2.97 | 3.41 | 4.00 | 4.32 | 4.59 | 4.91 |
| | Intraoral disintegration time | 5 | 6 | 7 | 8 | 9 | 10 |

As is clear from Table 5, in the case where calcium silicate and magnesium aluminometasilicate are added in combination, when the tableting pressure is increased, the hardness of the resultant tablet is increased without prolongation of the intraoral disintegration time thereof. The results show that a tablet containing lactose, powdered cellulose, magnesium aluminometasilicate, and a species selected from among the other silicic acid/silicic acid salts (i.e., a pharmaceutical composition according to the present invention) exhibits high hardness and excellent intraoral disintegrating property.
A tablet containing 10% ofloxacin serving as a pharmaceutically active ingredient was prepared, and the effect of the tableting pressure on the hardness and intraoral disintegration time of the tablet was evaluated.

### (Example 3)

Powdered cellulose (115.9 g), calcium silicate (27.3 g), magnesium aluminometasilicate (9.1 g), and crospovidone (27.3 g) were placed in a high-speed mixer, and were mixed together at 480 rpm for three minutes, to thereby prepare a powder mixture Y (179.6 g). Separately, powdered cellulose (2.3 g) and red ferric oxide (0.2 g) were mixed together by use of a tablet mill, to thereby prepare a powder mixture Z. The thus-prepared powder mixture Z (2.5 g), Orange Micron (0.9 g), ofloxacin (100.0 g), and the powder mixture Y (179.6 g) were mixed together by use of a high-speed mixer at 600 rpm for three minutes, to thereby prepare a powder mixture AA (283.0g). Subsequently, the powder mixture AA (283.0 g) and lactose (716.1 g) were placed in a V-type mixer, and were mixed together for 20 minutes, to thereby prepare a powder mixture BB. The thus-prepared powder mixture BB was subjected to tableting by use of an external-lubrication tableting machine (tableting pressure: 600 kgf, punch size: 7.0 mmφ), to thereby yield tablets, each having a weight of 110 mg (intraorally disintegrating pharmaceutical composition). In this case, magnesium stearate was employed as a lubricant.

### (Example 4)

The procedure of Example 3 was repeated, except that the tableting pressure was changed to 700 kgf, to thereby yield tablets (intraorally disintegrating pharmaceutical composition).

### (Example 5)

The procedure of Example 3 was repeated, except that the tableting pressure was changed to 800 kgf, to thereby yield tablets (intraorally disintegrating pharmaceutical composition).

### (Example 6)

The procedure of Example 3 was repeated, except that the tableting pressure was changed to 900 kgf, to thereby yield tablets (intraorally disintegrating pharmaceutical composition).

### (Example 7)

The procedure of Example 3 was repeated, except that the tableting pressure was changed to 1,000 kgf, to thereby yield tablets (intraorally disintegrating pharmaceutical composition).

The hardness and intraoral disintegration time of pharmaceutical compositions of the present invention containing 10% ofloxacin serving as a pharmaceutically active ingredient were measured. The results are shown in Table 6.

**Table 6**

| | Hardness (kp) | Intraoral disintegration time (seconds) |
|---|---|---|
| Example 3 | 4.1 | 8 |
| Example 4 | 4.2 | 9 |
| Example 5 | 4.8 | 10 |
| Example 6 | 5.5 | 13 |
| Example 7 | 6.4 | 15 |

The above results shown in Table 6 indicate that the pharmaceutical composition of the present invention containing 10% ofloxacin exhibits high hardness and excellent intraoral disintegrating property, regardless of the tableting pressure.

### [Industrial Applicability]

As is clear from the above-described Examples, the pharmaceutical composition of the present invention exhibits high hardness and excellent intraoral disintegrating property. Therefore, the pharmaceutical composition of the present invention is suitable for use as an intraorally disintegrating pharmaceutical composition.

## Claims

1. A pharmaceutical composition comprising a lactose and a powdered cellulose.

2. The pharmaceutical composition according to claim 1, which further comprises a disintegrant.

3. The pharmaceutical composition according to claim 2, wherein the disintegrant is one or more species selected from among low-substituted hydroxypropyl cellulose, crospovidone, sodium carboxymethyl starch, carmellose, and sodium croscarmellose.

4. The pharmaceutical composition according to claim 2 or 3, wherein the disintegrant is crospovidone and/or carmellose.

5. The pharmaceutical composition according to any one of claims 1 through 4, which further comprises a magnesium aluminometasilicate.

6. The pharmaceutical composition according to any one of claims 1 through 5, which further comprises one or more species selected from among silicic acid and silicic acid salts other than magnesium aluminometasilicate.

7. The pharmaceutical composition according to claim 6, wherein the one or more species selected from among silicic acid and silicic acid salts other than magnesium aluminometasilicate are calcium silicate and/or light anhydrous silicic acid.

8. The pharmaceutical composition according to any one of claims 1 through 7, which further comprises a pharmaceutically active ingredient.

9. The pharmaceutical composition according to any one of claims 1 through 8, wherein the total amount of the lactose and the powdered cellulose is 5 to 99.5 w/w% on the basis of the entire amount of the pharmaceutical composition.

10. The pharmaceutical composition according to any one of claims 1 through 9, wherein the amount of the powdered cellulose is 1 to 100 parts by weight on the basis of 100 parts by weight of the lactose.

11. The pharmaceutical composition according to any one of claims 2 through 10, wherein the amount of the disintegrant is 0.1 to 15 w/w% on the basis of the entire amount of the pharmaceutical composition.

12. The pharmaceutical composition according to any one of claims 5 through 11, wherein the amount of the magnesium aluminometasilicate is 0.05 to 5 w/w% on the basis of the entire amount of the pharmaceutical composition.

13. The pharmaceutical composition according to any one of claims 6 through 11, wherein the amount of the one or more species selected from among silicic acid and silicic acid salts other than magnesium aluminometasilicate is 0.05 to 5 w/w% on the basis of the entire amount of the pharmaceutical composition.

14. The pharmaceutical composition according to any one of claims 6 through 13, wherein the amount of the one or more species selected from among silicic acid and silicic acid salts other than magnesium aluminometasilicate is 0.01 to 20 parts by weight on the basis of 1 part by weight of the magnesium aluminometasilicate.

15. The pharmaceutical composition according to any one of claims 7 through 13, wherein the amount of the calcium silicate and/or the light anhydrous silicic acid is 0.01 to 20 parts by weight on the basis of 1 part by weight of the magnesium aluminometasilicate.

16. The pharmaceutical composition according to any one of claims 8 through 15, wherein the amount of the pharmaceutically active ingredient is 0.01 to 80 w/w% on the basis of the entire amount of the pharmaceutical composition.

17. The pharmaceutical composition according to any one of claims 1 through 16, which exhibits rapid disintegrating property.

18. The pharmaceutical composition according to any one of claims 1 through 16, which exhibits intraoral rapid disintegrating property.

19. The pharmaceutical composition according to any one of claims 1 through 18, which takes a form of tablet.

20. A pharmaceutical composition comprising a magnesium aluminometasilicate, and one or more species selected from among silicic acid and silicic acid salts other than magnesium aluminometasilicate.

21. The pharmaceutical composition according to claim 20, wherein the one or more species selected from among silicic acid and silicic acid salts other than magnesium aluminometasilicate are calcium silicate and/or light anhydrous silicic acid.

22. A method for increasing the hardness of a tablet, comprising adding a magnesium aluminometasilicate to a tablet.

23. The method according to claim 22, which further comprises adding, to the tablet, one or more species selected from among silicic acid and silicic acid salts other than magnesium aluminometasilicate.

24. A method for increasing the hardness of a tablet, comprising adding, to a tablet, a magnesium aluminometasilicate, and a calcium silicate and/or a light anhydrous silicic acid.

25. Use of the pharmaceutical composition according to any one of claims 1 through 16, for manufacturing an intraoral rapid disintegrating preparation.

26. Use according to claim 25, wherein the intraoral rapid disintegrating preparation is an intraoral rapid disintegrating tablet.
